Europäisches Patentamt

European Patent Office

Office européen des brevets

(19)

(11) **EP 0 779 024 B1**

(12) **EUROPEAN PATENT SPECIFICATION**

(45) Date of publication and mention
of the grant of the patent:
**16.11.2005 Bulletin 2005/46**

(51) Int Cl.$^7$: **A01H 5/10**, A01H 1/04

(21) Application number: **97200546.6**

(22) Date of filing: **30.09.1992**

(54) **A canola producing a seed with reduced glucosinolates and linolenic acid yielding an oil with low sulfur**

Canola mit Samen mit reduziertem Glucosinolat und Linolensäure, ein Öl ergebend mit niedrigem Schwefelgehalt

colza produisant une graine à teneur réduite en glucosinolates et en acide linolénique et permettant d'obtenir une huile à faible teneur en soufre

(84) Designated Contracting States:
**DE DK FR GB NL SE**

(30) Priority: **30.09.1991 US 767748**

(43) Date of publication of application:
**18.06.1997 Bulletin 1997/25**

(60) Divisional application:
**03076284.3 / 1 354 509**

(62) Document number(s) of the earlier application(s) in accordance with Art. 76 EPC:
**92921166.2 / 0 606 359**

(73) Proprietor: **Cargill Incorporated
Wayzata, Minnesota 55391 (US)**

(72) Inventors:
• **DeBonte, Lorin R.
Fort Collins, CO 80525 (US)**
• **Loh, Willie H.-T.
Minneapolis,MN 55417 (US)**
• **Fan, Zhegong X
Fort Collins, CO 80525 (US)**

(74) Representative: **Fisher, Adrian John
CARPMAELS & RANSFORD
43-45 Bloomsbury Square
London WC1A 2RA (GB)**

(56) References cited:
**EP-A- 0 323 753        WO-A-90/10380
WO-A-92/03919**

• **DATABASE FSTA INTERNATIONAL FOOD INFORMATION SERVICE (IFIS), FRANFURT/MAIN, DE THIES W ET AL: "(In Internationaler Rapskongress ((see FSTA (1978) 10 7N322)).)" XP002089868**
• **GRANDIS D: "STELLAR LOW LINOLENIC-HIGH LINOLEIC ACID SUMMER RAPE" CANADIAN JOURNAL OF PLANT SCIENCE, vol. 68, April 1988, pages 509-511, XP000671304**
• **B. R. STEFANSSON & Z.P. KONDRA: "Tower summer rape" CANADIAN JOURNAL OF PLANT SCIENCE, vol. 55, no. 1, January 1975, pages 343-344, XP002089867**
• **PREVOT A ET AL: "A NEW VARIETY OF LOW-LINOLENIC RAPESEED OIL;CHARACTERISTICS AND ROOM-ODOR TESTS" JOURNAL OF THE AMERICAN OIL CHEMISTS' SOCIETY, vol. 67, no. 3, March 1990, pages 161-164, XP000673031**
• **ROY N N ET AL: "PROSPECTS FOR THE DEVELOPMENT OF RAPESEED (B. NAPUS L.) WITH IMPROVED LINOLEIC AND LINOLENIC ACID CONTENT" PLANT BREEDING, vol. 98, 1987, pages 89-96, XP000671307**
• **DATABASE FSTA INTERNATIONAL FOOD INFORMATION SERVICE (IFIS), FRANFURT/MAIN, DE MORICE J: "Contribution of genetics and selection to improvement of production and product quality of the main oilseeds in temperate countries." XP002089869 & REVUE FRANCAISE DES CORPS GRAS, vol. 23, no. 11, 1976, pages 591-598, INRA, 35650 Le Rheu, France**

EP 0 779 024 B1

- **DATABASE FSTA INTERNATIONAL FOOD INFORMATION SERVICE (IFIS), FRANFURT/MAIN, DE KHAN A H ET AL: "Studies on the physico-chemical characteristics of a new genotype of autumn ((Brassica juncea cv.)) (zaid kharif raya)." XP002089870 & PAKISTAN JOURNAL OF SCIENTIFIC AND INDUSTRIAL RESEARCH, vol. 30, no. 12, 1987, pages 883-885, Oilseeds Res. Inst., Faisalabad, Pakistan**

Remarks:
The file contains technical information submitted after the application was filed and not included in this specification

**Description**

This invention relates to improved canola seeds, plants and oil having advantageous properties, that is, a low glucosinolates content and a very low $\alpha$-linolenic acid ($C_{18:3}$) content, which produce an oil with low sulfur content, improved sensory characteristics and oxidative stability.

1. Background

A need exists for an improved vegetable oil with a significantly extended shelf life and greater heat stability relative to generic canola oil and a positive nutritional contribution to animal, including human, diets.

Canola oil has the lowest level of saturated fatty acids of all vegetable oils. "Canola" refers to rapeseed (Brassica) which has an erucic acid ($C_{22:1}$) content of at most 2 percent by weight based on the total fatty acid content of a seed, preferably at most 0.5 percent by weight and most preferably essentially 0 percent by weight and which produces, after crushing, an air-dried meal containing less than 30 micromoles ($\mu$mol) per gram of defatted (oil-free) meal. These types of rapeseed are distinguished by their edibility in comparison to more traditional varieties of the species.

As consumers become more aware of the health impact of lipid nutrition, consumption of canola oil in the U. S. has increased. However, generic canola oil cannot be used in deep frying operations, an important segment of the food processing industry.

Canola oil extracted from natural and previously commercially useful varieties of rapeseed contains a relatively high (8%-10%) $\alpha$-linolenic acid content ($C_{18:3}$) (ALA). This trienoic fatty acid is unstable and easily oxidized during cooking, which in turn creates off-flavors of the oil (Gailliard, 1980, Vol. 4, pp. 85-116 In: Stumpf, P. K., ed., The Biochemistry of Plants, Academic Press, New York). It also develops off odors and rancid flavors during storage (Hawrysh, 1990, Stability of canola oil, Chapter 7, pp. 99-122 In: F. Shahidi, ed. Canola and Rapeseed: Production, Chemistry, Nutrition, and Processing Technology, Van Nostrand Reinhold, New York). One such unsatisfactory species heretofore has been Brassica napus, i.e., spring canola, a type of rapeseed.

It is known that reducing the $\alpha$-linolenic content level by hydrogenation increases the oxidative stability of the oil. Hydrogenation is routinely used to reduce the polyunsaturates content of vegetable oils, thereby increasing its oxidative stability. The food industry has used hydrogenation to raise the melting point of vegetable oils, producing oil-based products with textures similar to butter, lard and tallow. Trans isomers of unsaturated fatty acids are commonly produced during hydrogenation. However, the nutritional properties of trans fatty acids mimic saturated fatty acids, thereby reducing the overall desirability of hydrogenated oils (Mensink et al., New England J. Medicine N323:439-445, 1990; Scarth, et al., Can. J. Pl. Sci., 68:509-511, 1988). Canola oil produced from seeds having a reduced $\alpha$-linolenic acid content would be expected to have improved functionality for cooking purposes with improved nutritional value, and therefore have improved value as an industrial frying oil.

However, in general, very little variation exists for $\alpha$-linolenic acid content in previously known canola quality B. napus germplasm (Mahler et al., 1988, Fatty acid composition of Idao Misc. Ser. No. 125). Lines with levels of $\alpha$-linolenic acid lower than that of generic canola oil are known, but have sensory, genetic stability, agronomic or other nutritional deficiencies. For example, Rakow et al. (J. Am. Oil Chem. Soc., 50:400-403, 1973), and Rakow (Z. Pflanzenzuchtg, 69:62-82, 1973), disclose two $\alpha$-linolenic acid mutants, M57 and M364, produced by treating rapeseed with X-ray or ethylmethane sulfonate. M57 had reduced $\alpha$-linolenic acid while M364 had increased $\alpha$-linolenic acid. However, the instability of the fatty acid traits between generations was unacceptable for commercial purposes.

Brunklaus-Jung et al. (Pl. Breed., 98:9-16, 1987), backcrossed M57 and other rapeseed mutants obtained by mutagenic treatment to commercial varieties. $BC_0$ and $BC_1$ of M57 contained 29.4-33.3% of linoleic acid ($C_{18:2}$) and 4.9-10.8% of $\alpha$-linolenic acid ($C_{18:3}$). The oleic acid ($C_{18:1}$) content was not reported, but by extrapolation could not have exceeded 60%.

Four other lower $\alpha$-linolenic acid canola lines have been described. Stellar, reported by Scarth et al. (Can. J. Plant Sci., 68:509-511, 1988), is a Canadian cultivar with lower $\alpha$-linolenic acid (also 3%) derived from M57. Its $\alpha$-linolenic acid trait was generated by seed mutagenesis. S85-1426, a Stellar derivative with improved agronomic characteristics, also has lower (1.4%) $\alpha$-linolenic acid (Report of 1990 Canola/Rapeseed Strain Test A, Western Canada Canola Rapeseed Recommending Committee). IXLIN, another lower $\alpha$-linolenic acid (1.8%) line described by Roy et al. (Plant Breed., 98:89-96, 1987), originated from an interspecific selection. EP-A 323 753 (Allelix) discloses rape plants, seeds, and oil with reduced $\alpha$-linolenic acid content linked to limitations in the content of oleic acid, erucic acid, and glucosinolate.

Another nutritional aspect of rapeseed, from which canola was derived, is its high (30-55 $\mu$mol/g) level of glucosinolates, a sulfur-based compound. When the foliage or seed is crushed, isothiocyanate esters are produced by the action of myrosinase on glucosinolates. These products inhibit synthesis of thyroxine by the thyroid and have other anti-metabolic effects (Paul et al., Theor. Appl. Genet. 72:706-709, 1986). Brassica varieties with reduced glucosinolates content (<30 $\mu$mol/g defatted meal) were developed to increase the nutritional value of canola meal (Ste-

fansson et al., <u>Can. J. Plant Sci. 55</u>:343-344, 1975). Meal from an ultra-low glucosinolates line, BC86-18, has 2 μmol/g total glucosinolates and significantly improved nutritional quality compared to generic canola meal (Classen, Oral presentation, GCIRC Eighth International Rapeseed Congress, Saskatoon, Saskatchewan, July 9-11, 1991). Neither its fatty acid composition nor its seed glucosinolates profile is known.

There remains a need for an improved canola seed and oil with very low α-linolenic levels in the oil and low glucosinolates in the seed to significantly reduce the need for hydrogenation. The α-linolenic content of such a desirable oil would impart increased oxidative stability, thereby reducing the requirement for hydrogenation and the production of <u>trans</u> fatty acids. The reduction of seed glucosinolates would significantly reduce residual sulfur content in the oil. Sulfur poisons the nickel catalyst commonly used for hydrogenation (Koseoglu et al., Chapter 8, pp. 123-148, <u>In</u>: F. Shahidi, ed. Canola and Rapeseed: Production, Chemistry, Nutrition, and Processing Technology, Van Nostrand Reinhold, New York, 1990). Additionally, oil from a canola variety with low seed glucosinolates would be less expensive to hydrogenate.

2. <u>Summary of the Invention</u>

This invention comprises a canola (Brassica napus) plant according to claim 1.

This invention further comprises a seed according to claim 2, the progeny of such seed and oil of such a seed possessing the quality traits of interest.

3. <u>Detailed Description of the Invention</u>

A spring canola (<u>Brassica napus</u> L.) variety was developed with improved sensory characteristics and oxidative stability in the seed oil. This variety, designated IMC 01, has very low levels of α-linolenic acid ($CH_3CH_2CH=CHCH_2CH=CHCH_2CH=CH(CH_2)_7COOH$) in the seed oil and very low levels of glucosinolates in the seed. The oil produced from the seed of this variety has very low levels of sulfur and was shown to have significantly improved sensory characteristics over generic canola oils. The IMC 01 is a line in which these traits have been stabilized for both the generation to which the seed belongs and that of its parent generation. Particularly desirable lines of this invention from an agronomic point of view can be derived by conventionally crossing lines producing seeds meeting the definitions of this invention with agronomically well-proven lines such as Westar.

In the context of this disclosure, a number of terms are used. As used herein, a "line" is a group of plants that display little or no genetic variation between individuals for at least one trait. Such lines may be created by several generations of self-pollination and selection, or vegetative propagation from a single parent using tissue or cell culture techniques. As used herein, the terms "cultivar" and "variety" are synonymous and refer to a line which is used for commercial production. "Stability" or "stable" means that with respect to the given component, the component is maintained from generation to generation and, preferably, at least three generations at substantially the same level, e.g., preferably ± 15%, more preferably ± 10%, most preferably ± 5%. The stability may be affected by temperature, location, stress and the time of planting. Comparison of subsequent generations under field conditions should produce the component in a similar manner. "Commercial Utility" is defined as having good plant vigor and high fertility, such that the crop can be produced by farmers using conventional farming equipment, and the oil with the described components can be extracted from the seed using conventional crushing and extraction equipment. To be commercially useful, the yield, as measured by both seed weight, oil content, and total oil produced per acre, is within 15% of the average yield of an otherwise comparable commercial canola variety without the premium value traists grown in the same region. "Agronomically elite" means that a line has desirable agronomic characteristics such as yield, maturity, disease resistance, standability. The amount of fatty acids, such as oleic and linolenic acids, that are characteristic of the oil is expressed as a percentage of the total fatty acid content of the oil. "Saturated fatty acid" refers to the combined content of palmitic acid and stearic acid. "Polyunsaturated fatty acid" refers to the combined content of linoleic and α-linolenic acids. The term "shortening" refers to an oil that is a solid at room temperature. The term "room odor" refers to the characteristic odor of heated oil as determined using the room-odor evaluation method described in Mounts (<u>J. Am. Oil Chem. Soc.</u>, 56:659-663, 1979). "Generic canola oil" refers to a composite oil extracted from commercial varieties of rapeseed currently known as of the priority date of this application, which varieties generally exhibited at a minimum 8-10% α-linolenic acid content, a maximum of 2% erucic acid and a maximum of 30 μmol/g total glucosinolate level. The seed from each growing region is graded and blended at the grain elevators to produce an acceptably uniform product. The blended seed is then crushed and refined, the resulting oil being sold for use. Table A shows the distribution of canola varieties seeded as percentage of all canola seeded in Western Canada in 1990.

TABLE A:

| Distribution of Canola Varieties Grown in Western Canada in 1990 | |
| --- | --- |
| Canola Variety | Percent of Seeded Area |
| *B. campestris* | |
| Candle | 0.4 |
| Colt | 4.4 |
| Horizon | 8.5 |
| Parkland | 2.5 |
| Tobin | 27.1 |
| *B. napus* | |
| Alto | 1.1 |
| Delta | 0.9 |
| Global | 0.9 |
| Legend | 18.2 |
| Pivot | 0.1 |
| Regent | 0.5 |
| Stellar | 0.2 |
| Tribute | 0.4 |
| Triton | 0.7 |
| Triumph | 0.2 |
| Westar | 29.5 |
| Others | 4.4 |
| Source: Quality of Western Canadian Canola - 1990 Crop Year. Bull. 187, DeClereg et al., Grain Research Laboratory, Canadian Grain Commission, 1404-303 Main Street, Winnipeg, Manitoba, R3C 3G8. | |

IMC 01 is a very low $\alpha$-linolenic acid (<4.1% $C_{18:3}$) line selected during an extensive germplasm screening effort. Its parentage is unknown. IMC 01 was self-pollinated and selected for low $\alpha$-linolenic acid (<4.1%) over four consecutive generations. At each generation, seeds from individually pollinated plants were analyzed for fatty acid composition. Data showed no genetic segregation for $\alpha$-linolenic acid content over five generations of self-pollinations (Table I). Breeder seed was derived from a bulk seed increase of selected plants from the fourth self-crossed generation.

TABLE I:

| Fatty Acid Composition of IMC 01 Over Five Generations | | | | | |
| --- | --- | --- | --- | --- | --- |
| DATE OF ANALYSIS | PERCENT COMPOSITION | | | | |
| | $C_{16:0}$ | $C_{18:0}$ | $C_{18:1}$ | $C_{18:2}$ | $C_{18:3}$ |
| 11/87 | 4.1 | 1.9 | 64.1 | 25.7 | 1.9 |
| 8/88 | 4.6 | 2.3 | 72.6 | 14.4 | 2.0 |
| 1/89 | 4.9 | 1.5 | 60.4 | 25.8 | 2.5 |
| 4/89 | 4.8 | 1.8 | 64.3 | 21.4 | 4.0 |
| 10/89 | 4.3 | 2.1 | 64.1 | 24.8 | 2.0 |

IMC 01 was planted in replicated field trials in North Dakota, South Dakota, Minnesota, Washington, Idaho and Montana in 1989 and 1990, under both irrigated and nonirrigated conditions. These tests showed that the $\alpha$-

linolenic acid content of IMC 01 was sensitive to temperature (Table II). This was further supported by growing IMC 01 under controlled temperature conditions in growth chambers. Whether or not the observed temperature sensitivity of IMC 01 is common to other low α-linolenic acid canola lines is unknown.

A temperature effect on fatty acid compositions has been widely reported in plants, especially oilseed crops (Rennie et al., J. Am. Oil Chem. Soc., 66:1622-1624, 1989). These reports describe general temperature effects on fatty acid composition.

Changes in fatty acid content in seed oil under cool temperatures have been documented in plants such as soybean, peanut and sunflower (Neidleman, In: Proceedings of the World Conference on Biotechnology for the Fats and Oils Industry, Applewhite, T. H., ed., pp. 180-183. Am. Oil. Chem. Soc. 1987).

TABLE II:

| α-Linolenic acid Content of IMC 01 in Production in 1990 | | |
|---|---|---|
| PRODUCTION REGION | $\bar{X}$ TEMPERATURE DURING SEED MATURATION | α-LINOLENIC ACID CONTENT |
| Eastern Washington | 74°F | 1.9% |
| Eastern washington | 74°F | 2.0% |
| Northern Idaho | 70°F | 3.0% |
| Northern Idaho | 67°F | 2.9% |
| Eastern Idaho | 62°F | 3.5% |
| Southern Montana | 66°F | 4.1% |
| Central Montana | 67°F | 4.0% |

In addition to very low α-linolenic acid, IMC 01 is also characterized by very low levels of glucosinolates. Glucosinolates are sulfur-based compounds common to all Brassica seeds. Glucosinolates exist in aliphatic or indolyl forms. Aliphatic glucosinolates can be analyzed via gas chromatography (GC) (Daun, Glucosinolate analysis of rapeseed (canola), Method of the Canadian Grain Commission, Grain Research Laboratory, Canadian Grain Commission, Winnipeg, 1981). Indolyl glucosinolates have only recently been analyzed via high performance liquid chromatograph (HPLC). Prior to the adoption of the HPLC method, total glucosinolates were calculated by multiplying the aliphatic glucosinolates by a correction factor. Canola quality in the seed is defined as having <30 μmol/g of glucosinolates in the defatted meal.

IMC 01 and Westar were tested in five locations in southeastern Idaho in 1990. Three of the locations were irrigated (I) and two were dryland (D) conditions. Table IIIa shows the difference in total aliphatic glucosinolate between IMC 01 and Westar grown at these locations. The aliphatic glucosinolate values are reported as μmol/gm of defatted meal.

The aliphatic glucosinolate content of IMC 01 by location was consistently lower and more stable than that of Westar at all locations tested. The average glucosinolate contents of IMC 01 was 4.9 μMol/gm while Westar was 13.3 μmol/gm. A Least Significant Difference (LSD) test was used to determine if the two were significantly different at all lcoations. IMC 01 was found to be significantly different from Westar at a level of $P<0.05$.

HPLC analysis of IMC 01 vs Westar, the most widely grown spring canola variety in North America, shows that IMC 01 has much lower levels of aliphatic glucosinolates (Table III). No significant differences exist for indolyl glucosinolates. Glucosinolates content is also subject to environment influence, e.g., sulfur fertility and drought stress. However, IMC 01 consistently had the lowest and the most stable aliphatic glucosinolates levels at all locations tested (Table IV). The locations tested differ in altitude, temperature, fertility, irrigation, and other cultural practices. Among the low α-linolenic canola lines for which glucosinates analysis have been performed, IMC 01 has the lowest level of total seed glucosinolates (Table V).

TABLE III:

| Glucosinolates Profiles of IMC 01 and Westar Varieties | | |
|---|---|---|
| GLUCOSINOLATES (μmol/g) | IMC 01 | WESTAR |
| 3-butenyl | 1.2 | 4.2 |
| 4-pentenyl | 0.1 | 0.2 |
| 2-OH-3-butenyl | 3.1 | 7.0 |

TABLE III:   (continued)

| Glucosinolates Profiles of IMC 01 and Westar Varieties | | |
| --- | --- | --- |
| GLUCOSINOLATES (µmol/g) | IMC 01 | WESTAR |
| 2-OH-4-pentenyl | 0.9 | 0.4 |
| Total Aliphatics | 5.3 | 11.8 |
| 4-OH-3-indolylmethyl | 6.2 | 6.1 |
| 3-indolylmethyl | 0.8 | 1.0 |
| 4-methoxyindolyl | 0.1 | tr |
| 1-methoxyindolyl | 0.1 | 0.2 |
| Total Indolyls | 7.2 | 7.3 |
| Total Glucosinolates | 12.5 | 19.1 |

TABLE IV:

| Aliphatic Glucosinolates of IMC 01 and Westar over Different Environments in Southeastern Idaho | | |
| --- | --- | --- |
| LOCATION* | ALIPHATIC GLUCOSINOLATES CONTENT (µmol/g) | |
| | IMC 01 | WESTAR |
| Newdale - I | 4.7 | 13.0 |
| Soda Springs - D | 6.3 | 9.9 |
| Tetonia - D | 5.0 | 13.5 |
| Tetonia -I | 3.7 | 13.3 |
| Shelley - I | 5.0 | 17.0 |
| Average | 4.9 | 13.3 |
| Standard Deviation | 0.93 | 2.51 |

*I = Irrigated, D = Dryland

TABLE V:

| Glucosinolates Content of Low α-Linolenic acid Canola Varieties and Westar | | | |
| --- | --- | --- | --- |
| GLUCOSINOLATES (µmol/g) | ALIPHATIC | INDOLYL | TOTAL |
| IMC 01 | 5.3 | 7.2 | 12.5 |
| Stellar | 5.2 | 19.5 | 24.7 |
| S85-1426 | 7.9 | 13.4 | 21.3 |
| Westar | 11.8 | 7.3 | 19.1 |

IMC 01 was produced, using normal production practices for spring canola, in Idaho and North Dakota in 1988, in Idaho, Washington State and Montana in 1989, in Idaho, Washington State, Montana, Oregon, and Wyoming in 1990. When grown in suitable environments, where the average daily temperature (high temperature plus low temperature divided by 2) exceeds 20°C, the oil contains <4.1% α-linolenic acid. As an example, a normal fatty acid profile was produced at Casselton, North Dakota. The crop produced in Ashton, Idaho, was subject to extremely cool conditions and had higher levels of α-linolenic acid. The crops obtained from the field tests were crushed and processed to produce refined, bleached and deodorized (RBD) canola oil at the Protein, Oil, Starch (POS) Pilot Plant in Saskatoon, Saskatchewan. A method of bleaching canola oil is provided in the AOCS' Recommended Practice Cc 8a-52 (AOCS Methods and Standard Practices, 4th Edition (1989)). A method for the refining of crude oils is provided in the AOCS Practice Cc 9a-52 (AOCS Methods and Standard Practices, 4th Edition (1989)). The oils were tested at the Vegetable Oil Research Laboratory, U.S.D.A./Northern Regional Research Center, for organoleptic and sensory characteristics.

Testing to assure that desirable sensory characteristics are obtained in the Brassica napus variety was essential. The evaluation of odors has been conducted in a variety of ways on low α-linolenic acid canola oils. The testing methods are based on the fact that vegetable oils emit characteristic odors upon heating. For example, Prevot et al. (J. Amer. Oil Chemists Soc. 67:161-164, 1990) evaluated the odors of a French rapeseed, "Westar", and "low linolenic" canola oils in a test which attempted to reproduce domestic frying conditions. In these evaluations the test oils were used to fry potatoes and the odors were evaluated by a test panel. The odor tests showed that the "low linolenic" (approximately 3%) line had a significantly higher (more acceptable) odor score than the French rapeseed and "Westar" lines, which were very similar to each other. Eskin et al. (J. Amer. Oil Chemists Soc. 66:1081-1084, 1989) evaluated the odor from canola oil with a low linolenic acid content, a laboratory deodorized sample, and a commercially deodorized sample by sniffing in the presence of the oil itself. These studies demonstrated that a reduction in the linolenic acid content of canola oil from 8-9% to 1.6% reduced the development of heated odor at frying temperatures. However, the odor of the low linolenic acid oil was still unacceptable when heated in air to a majority of the panelists, suggesting that low linolenic acid alone is not sufficient to guarantee acceptable odor.

Mounts (J. Am. Oil Chem. Soc., 56:659-663, 1979) describe a distinct room-odor evaluation method that is used to reproducibly assess the odor characteristics of a cooking oil upon heating. This is the evaluation method of choice owing to its reproducibility and its approximation of odors emitted upon heating the oil. In this method, the oil is heated in a separate chamber and the odor pumped into the room containing the trained evaluators. As noted elsewhere, where the term "room-odor" is used herein, it refers to this method of Mounts. This method is distinct from earlier described tests where the oil and evaluator are within the same room. Such same room testing is referred to as "uncontrolled bench top odor tests" and is considered less accurate and less reliable than the Mounts' room odor evaluation method.

The room-odor characteristics of cooking oils can be reproducibly characterized by trained test panels in room-odor tests (Mounts, J. Am. Oil Chem. Soc. 56:659-663, 1979). A standardized technique for the sensory evaluation of edible vegetable oils is presented in AOCS' Recommended Practice Cg 2-83 for the Flavor Evaluation of Vegetable Oils (Methods and Standard Practices of the AOCS, 4th Edition (1989)). The technique encompasses standard sample preparation and presentation, as well as reference standards and method for scoring oils. When heated, generic canola oil has reduced stability and produces offensive room odors. Refined-Bleached-Deodorized (RBD) canola oil is characterized by a fishy flavor in such tests. This characteristic is commonly ascribed to its high polyunsaturated fatty acid content, particularly α-linolenic acid, relative to other vegetable oils. The individual fragrance notes (odor attributes) of the oils are evaluated by Least Significant Difference Analysis. Notes which differ by greater than 1.0 can be reproducibly measured by a sensory panel. In these tests, IMC 01 oil expressed significantly reduced levels of the offensive odors (Table VI).

TABLE VI:

| Room Odor Intensity of IMC 01 and Generic Canola Oil | | |
|---|---|---|
| ODOR ATTRIBUTES | IMC 01 | GENERIC CANOLA OIL |
| Overall | 4.6[a] | 7.4[b] |
| Fried Foods | 1.8 | 3.5 |
| Doughy | 1.0 | 0 |
| Fishy | 0[a] | 5.5[b] |
| Burnt | 0[a] | 0.9[b] |
| Acrid | 0[a] | 2.3[b] |
| Woody/Cardboard | 1.9 | 0 |
| Hydrogenated | 0 | 0 |
| Pastry/Sugary | 0 | 0 |
| Waxy | 0 | 0 |
| Chemical | 0 | 0 |
| 0 = None; 10 = Strong. Scores with different superscript letters are significantly different (P <0.05). Least Significant Difference for individual odor notes is 1.0. Differences greater than 1.0 can be reproducibly measured by room-odor analysis. | | |

Due to its relatively low stability, canola oil is often hydrogenated for frying. However, hydrogenation produces a characteristic (hydrogenated) room odor which is unacceptable to food manufacturers. Surprisingly, hydrogenated IMC 01 oil also has reduced levels of the characteristic hydrogenated room odor (Table VII). Table VII shows that the overall room-odor intensity of hydrogenated IMC 01 is significantly less than that of hydrogenated generic oil as indicated by a difference is scores of greater than 1.0 in standardized flavor evaluation trials.

TABLE VII:

| Room Odor Intensity and Individual Odor Descriptions for Hydrogenated Canola Oils | | | |
|---|---|---|---|
| ODOR ATTRIBUTE | HYDROGENATED, GENERIC CANOLA SHORTENING | HYDROGENATED IMC 01 SHORTENING(2% $\alpha$-LINOLENIC ACID) | HYDROGENATED IMC 01 SHORTENING (6.8% $\alpha$-LINOLENIC ACID) |
| Overall Intensity | 6.6[b] | 3.8[a] | 3.9[a] |
| Fried Food | 2.7 | 1.1 | 1.5 |
| Doughy | 1.2 | 0.6 | 0.8 |
| Fishy | 0.6 | 0 | 0 |
| Burnt | 0.5 | 0 | 0 |
| Acrid | 0.8 | 0 | 0 |
| Hydrogenated | 3.2 | 1.8 | 2.3 |
| Waxy<br>Other | 0.5<br>4.5<br>rubbery<br>flowery<br>weedy<br>soapy | 0.6<br>2.4<br>fruity<br>smoky | 0.<br>2.8<br>fruity<br>flowery<br>sweet<br>pastry |
| 0 - None; 10 = Strong. Scores with different superscript letters are significantly different (P <0.05). Least Significant Difference for individual odor notes is 1.0. Differences greater than 1.0 can be reproducibly measured by room-odor analysis. | | | |

IMC 01 produces an oil which has improved sensory characteristics. Such improvements have been predicted for low $\alpha$-linolenic acid canola oils (Ulrich et al., J. Am. Oil Chem. Soc., 8:1313-1317, 1988). However, the improved sensory characteristics of IMC 01 appears not to be related solely to its low $\alpha$-linolenic acid content. Surprisingly, IMC 01 canola oils with both high and low levels of $\alpha$-linolenic acid showed similar degrees of improvement. Sensory tests have shown that IMC 01 oil maintains its improved quality at both 2% and 6.8% $\alpha$-linolenic acid.

The very low glucosinolates characteristic of IMC 01 seed is believed to contribute to the improved sensory characteristic of IMC 01 oil. Glucosinolates in the seed are converted to sulfur compounds. Most of the sulfur breakdown products remain in the meal, but some inevitably contaminate the oil. Lower levels of glucosinolates in the seed are believed to result in lower sulfur content in the oil, and this is believed to reduce the objectionable odor characteristics of canola oil (Abraham et al., J. Am. Oil Chem. Soc., 65:392-395, 1988). An analysis of the sulfur content of IMC 01 oil and several generic canola oils has been performed. IMC 01 oil has approximately one-third the sulfur content of leading generic canola oils (Table VIII).

TABLE VIII:

| Sulfur Content of Canola Oils | |
|---|---|
| Canola Oils | Sulfur Content |
| Acme Brand Canola Oil | 3.8 ppm |
| Hollywood Brand Canola Oil | 3.8 ppm |
| Puritan Brand Canola Oil | 3.9 ppm |
| IMC 01 Canola Oil | 1.3 ppm |

The biochemical, molecular and genetic mechanisms responsible for the room-odor quality of vegetable oils are not fully understood. Improvements in vegetable oil processing technology, i.e., preferential removal of sulfur during processing, less abusive oil extraction procedures, minimal processing, gentler deodorization, etc., may improve the overall quality of vegetable oils, including both sensory and functional characteristics (Daun et al., J. Am. Oil Chem. Soc., 53:169-171, 1976). IMC 01 will benefit from any such processing improvements, and will maintain its improved sensory characteristics over generic canola oil under equivalent processing conditions.

IMC 01 is true breeding as are its progeny. The traits responsible for reduced $\alpha$-linolenic acid and reduced total glucosinolates in the seed which yield an oil low in sulfur having improved sensory characteristics have a genetic basis. The data presented herein show that these traits are stable under different field conditions. These traits can be removed from the IMC 01 background and are transferred into other backgrounds by traditional crossing and selection techniques.

Crosses have been made with IMC 01 as one parent to demonstrate that the superior IMC 01 quality/sensory traits are transferred along with the superior agronomic traists of another parent such as the Canadian canola line, Westar, into descendents. The parent to which IMC 01 is crossed is chosen on the basis of desirable characteristics such as yield, maturity, disease resistance, and standability. Conventional breeding techniques employed in such crossings are well known by those skilled in the art. Thus, a method of using the IMC 01 Brassica napus is to cross it with agronomically elite lines to produce plants yielding seeds having the characteristics listed above.

The general protocol is:

a. cross IMC 01 to a selected parent;
b. produce a "gametic array" using microsphores of the $F_1$ plants to produce dihaploid (DH) individuals;
c. field trial $DH_2$ individuals for yield and select from IMC 01 $\alpha$-linolenic acid and glucosinolate levels; and
d. test selected individuals for oil quality using RBD oil.

Example 3 is a specific example of such work to develop descendents to IMC 01 which retain the desirable quality traits. The data of Example 3 show that the quality traits of IMC 01 are heritable in such crosses.

The present invention is further defined in the following Examples, in which all parts and percentages are by weight and degrees are Celsius, unless otherwise stated. It should be understood that this Example, while indicating preferred embodiments of the invention, is given by way of illustration only.

EXAMPLE 1

IMC 01, originally designated DNAP #336, was grown in a greenhouse in Cinnaminson, New Jersey, over several seasons to select for a stable, very low $\alpha$-linolenic line. Day/night temperatures from August through December in the greenhouse averaged 80°F/65°F with fluctuations of $\pm$ 5°F, 75°F/65°F from January through April, and 85°F/ 65°F from March through July. The plants were grown in 1-gallon pots under natural day length, except from October through May when the plants received 14 hours of supplemental lighting. Flowering racemes were covered with paper bags to prevent cross-pollination, and gently shaken to induce seed set. Watering was decreased as pods reached maturity.

For field testing, IMC 01 was planted in multi-location trials and production plots in Montana, Idaho and Washington. The trials were planted in a completely randomized block design with four replications. Each block contained eight plots of 6 meters by 8 rows. IMC 01 was also planted in large acreages (>25 acres) according to standard agronomic procedures for spring canola production, with a minimum ½-mile isolation from other Brassica napus crops. Depending on location, the fields were planted in April or May, and harvested in August or September. Plantings were made on dryland, following both fallow or recrop, or under irrigation. Mature pod samples were taken following swathing for chemical analysis.

For fatty acid analysis, 10-50 seed samples were ground in 15-mL polypropylene tubes and extracted in 1.2 mL 0.25 N KOH in 1:1 ether/methanol. The sample was vortexed for 10 sec and heated for 60 sec and in 60°C water bath. Four mL of saturated NaCl and 2.4 mL of iso-octane were added, and the mixture was vortexed again. After phase separation, 600 $\mu$L of the upper organic phase was pipetted into individual vials and stored under nitrogen. One $\mu$L sample was injected into a Supelco SP-2330 fused silica capillary column (0.25 mm ID, 30 m length, 0.20 $\mu$m df, Bellfonte, PA).

The gas chromatograph was set at 180°C for 5.5 min, then programmed for a 2°C/min increase to 212°C, and held at this temperature for 1.5 min. Chromatography settings were: Column head pressure - 15 psi, Column flow (He) - 0.7 mL/min, Auxiliary and Column flow - 33 mL/min, Hydrogen flow = 33 mL/min, Air flow - 400 mL/min, Injector temperature - 250°C, Detector temperature - 300°C, Split vent - 1/15.

A standard industry procedure for HPLC analysis of glucosinolates was used to analyze the glucosinolates composition of the seed (Daun et al., In: Glucosinolate Analysis of Rapeseed (Canola). Method of the Canadian Grain Commission, Grain Research Laboratory, 1981).

IMC 01 seed was harvested and processed to produce refined, bleached and deodorized (RBD) oil. Some oil was hydrogenated after refining, bleaching and deodorization, then redeodorized.

Before extraction, the seed was tempered to adjust the moisture content to 9% and flaked to 0.38 to 0.64 cm in a ribbon blender. The flakes were cooked in a stack cooker at 82.8°C for 30 min (8.5% moisture) and pre-pressed with vertical and horizontal bar spacings set to 0.031 cm, vertical shaft speed at 40 rpm and horizontal shaft at 25 rpm. The press cake was extracted in a Crown Model 2 extractor at 37.3 kg and hexane extracted with a 2:1 solvent to solids ratio.

The crude oil was alkali refined at 65°C-70°C for 30 min with 0.2% to 85% phosphoric acid, then mixed with sodium hydroxide to neutralize free fatty acids. Soaps were removed with a water wash (65°C water, 5 min) and the oils bleached with .75% each by weight of Clarion and Acticil bleaching earths for 30 min to remove color bodies. The resulting oil contained no peroxides, 0.08% free fatty acids, and had a Gardner color of 10-.

The oil was continuously deodorized at 265°C at 300 kg/h. The steam rate was 1% of feed rate. The deodorized oil was preheated to 68-72°C prior to deaeration. RBD oil was stored in food grade plastic drums or pails at 4°C under nitrogen prior to testing.

For hydrogenation, RBD oil was heated to 350°F under vacuum in a stainless steel pressure reactor. A 0.5% sulfur-poisoned nickel catalyst, Englehardt SP-7, was added to the oil at 80.1°C, and hydrogen gas was introduced at 40 psi. Periodic samples were analyzed until an oil with a 30.5°C melting point was achieved. The hydrogenated oil was redeodorized and stored by the methods described previously.

The RBD and hydrogenated oil samples were analyzed for room-odor characteristics by a trained test panel in comparison with a generic, commercially available RBD canola oil (Proctor & Gamble) and generic, commercially available hydrogenated canola shortening as described previously. The testing protocol used is described in Mounts (J. Am. Oil Chem. Soc. 56:659-663, 1979) which is hereby incorporated by reference. The testing controlled for temperature of the oil, distance from the oil and room volume, and required that the oil was heated in a separate chamber and pumped into the room containing the trained panelist.

Specifically, room odor profiles of IMC 01 and a generic canola oil were obtained as follows:

A. Room Odor Protocol

A 150 mL sample of the selected oil was heated to 190°C for 30 min before the start of each panel test. The oil was maintained at this temperature throughout each session. For each session a fresh oil sample was used.

Panelists visited each odor room for approximately 15 sec. A five min rest was required between visits. Visitation to each odor room was randomized among the panelists.

The trained panelists judged the room odor for intensity of odor, quality of odor, and odor attributes. The intensity was ranked as: 0-4 weak, 5-7 moderate, and 8-10 strong. The quality of odor was judged as: 0-1 bad, 2-3 poor, 4-6 fair, 7-8 good, and 9-10 excellent. The odor attributes were ranked as: 0-1 bland, 2-4 weak, 5-7 moderate, and 8-10 strong. The flavor attributes were fried, painty, fishy, hydrogenated, burnt, cardboard, metallic, rubbery, waxy, buttery, and nutty.

B. Generic Oil - IMC 01 Profile Comparison

A generic, commercially available canola oil (Proctor & Gamble) was used in the IMC room odor tests as the standard or generic canola oil. In a comparative test, the standard canola oil was significantly (P<0.05) higher in room odor intensity than IMC 01 (Table IX). The standard canola oil odor was of "moderate" intensity while the IMC 01 was considered "weak". The overall quality of the IMC 01 room odor was significantly (P<0.05) better than the standard canola oil. The standard canola oil had significantly (P<0.05) higher intensities for fried, painty, and cardboard odors than the IMC 01 oil.

Table IX:

| Room Odor Profile of Generic (Proctor & Gamble) and IMC 01 Oil | | |
|---|---|---|
| Evaluation* | IMC 01 | Generic |
| A. Intensity | 3.4[a] | 5.2[b] |
| B. Quality | 5.8[a] | 4.9[b] |
| C. Odor Attributes | | |

* The "intensity" was ranked as: 0-4 weak, 5-7 moderate, and 8-10 strong. The "quality" of odor was judged as: 0-1 bad, 2-3 poor, 4-6 fair, 7-8 good, and 9-10 excellent. The "odor attributes" were ranked as: 0-1 bland, 2-4 weak, 5-7 moderate, and 8-10 strong. Scores with different superscript letters are significantly different (P <0.05).

Table IX: (continued)

| Room Odor Profile of Generic (Proctor & Gamble) and IMC 01 Oil | | |
|---|---|---|
| Evaluation* | IMC 01 | Generic |
| Fried | 1.9[a] | 2.9[b] |
| Painty | 0.4 | 1.3 |
| Fishy | 0.8[a] | 1.9[b] |
| Hydrogenated | 1.1 | 0.6 |
| Rancid | 0.7 | 0.9 |
| Burnt | 0.8 | 1.4 |
| Cardboard | 0.1[a] | 1.5[b] |
| Metallic | 0.5 | 0.1 |
| Rubbery | 0.0 | 0.0 |
| Waxy | 0.6 | 0.0 |
| Buttery | 0.7 | 0.3 |

* The "intensity" was ranked as: 0-4 weak, 5-7 moderate, and 8-10 strong. The "quality" of odor was judged as: 0-1 bad, 2-3 poor, 4-6 fair, 7-8 good, and 9-10 excellent. The "odor attributes" were ranked as: 0-1 bland, 2-4 weak, 5-7 moderate, and 8-10 strong. Scores with different superscript letters are significantly different (P <0.05).

Pilot plant-processed samples of Example 2 generic canola (low erucic acid rapeseed) oil and oil from IMC 01 canola with the fatty acid compositions modified by mutation breeding and/or hydrogenation were evaluated for frying stability α-linolenic acid contents were 10.1% for generic canola oil, 1.7% for canola modified by breeding (IMC 01) and 0.8% and 0.7% for IMC 01 oils modified by breeding and hydrogenation. The IMC 01 modified oils had significantly (P <0.05) less room odor intensity that the generic canola oil after initial heating tests at 190°C as judged by a sensory panel under conditions of AOCS Cg 2-83. The generic canola oil had significantly higher intensities for fishy, burnt, rubbery, smoky, and acrid odors than the modified oils. Foam heights of the modified oils were significantly (P <0.05) less than those of the generic oil after 20, 30 and 40 hrs of heating and frying at 190°C. The flavor quality of french fried potatoes was significantly (P <0.05) better for all the potatoes fried in modified oils than those fried in generic canola oil. The potatoes fried in generic canola oil were described by the sensory panel as fishy. No off-flavors were detected in potatoes fried in the modified oils.

EXAMPLE 3

This Example demonstrates that the traits of very low α-linolenic acid and very low glucosinolate content are transferred to IMC 01 progeny.

```
Year 1    IMC 01                    X      Westar
          ALA Content:  2.5%               ALA Content:  8.5%
          Glucosinolates:  12 μmol/g  Glucosinolates:  21 μmol/g

                                  ↓

                                 F₁     ←       Gametic Array

                                  ↓

Year 2                Preliminary Field Trial (DH₁ Seed)
                               Idaho


                                  ↓     ←        Select Line HW3.001

Year 3                Stage I Field Trial (DH₂ Seed)
                      Location:  Idaho
                      ALA Content:  1.6%

                      ↓                    ↓


                      Greenhouse (DH₃)     Isolation Tents (DH₃)
                      Single Plants        California
                      ALA Range:  2.5-2.8% ALA Content:  2.5%
                                           Glucosinolates: 10 <μmole/gm

                                                 ↓
Year 4
                                   Pre-Production Increase (DH₄)
                                   Idaho
                                   ALA 2.1%
```

The pre-production will be crushed and the oil refined for quality.

Once a canola line has been stabilized, fully conventional methods of plant biotechnology, breeding and selection are used to further enhance, for example, the agronomic properties of the resultant line in order to improve important factors such as yield, hardiness, etc. Such techniques are also well known and include, e.g., somaclonal variation, seed mutagenesis, anther and microspore culture, protoplast fusion, etc. See, e.g., Brunklaus-Jung et al., Pl. Breed., 98:9-16, 1987; Hoffmann et al., Theor. Appl. Genet., 61, 225-232 (1982), each herein incorporated by reference).

A deposit of seed designated IMC 01 has been made in the American Type Culture Collection (ATCC) depository (Rockville, MD 20852) and bears accession number ATCC 40579. The deposit was made on 2 March 1989 under conditions complying with the requirements of the Budapest Treaty.

## Claims

1. A Canola (*Brassica napus*) plant comprising
   seed having a total glucosinolates content of 18 μmol or less/g of defatted, air-dried meal;
   the seed yielding oil having an α-linolenic acid content of 7% or less relative to total fatty acid content of said seed and a sulfur content of less than or equal to 3.0 ppm; and
   the plant belonging to a line in which these traits have been stabilized for at least three generations.

2. The seed produced by the plant of Claim 1.

3. The seed produced by the plant of Claim 1 wherein the total glucosinolates content is 15 μmol or less/g of defatted, air-dried meal.

4. The oil of the seed produced by the plant of Claim 1.

**5.** A *Brassica napus* plant wherein at least one parent was the plant of Claim 1.

**6.** Progeny of the plant of Claims 1 or 5.

**7.** A plant produced from the crossing of a canola (*Brassica napus*) plant according to claim 1, with an agronomically elite variety of *Brassica napus*, the plant yielding a seed having a total glucosinolates content of 18 μmol or less/ g of defatted, airdried meal, and an extractable oil having (1) an α-linolenic acid content of 7% or less relative to total fatty acid content of said seed, and (2) a sulfur content of less than or equal to 3.0 ppm

**8.** The plant of Claim 7, wherein the agronomically elite parent is the Canadian canola line, Westar.

**9.** The plant of any one of claims 1, 7 or 8, wherein the seeds have a total glucosinolate content of 12.5 μmol or less/ g of defatted, air-dried meal.

**10.** The plant of any one of claims 1, 7 or 8, wherein the seeds have an aliphatic glucosinolate content of from 3.7 μmol/g to 6.3 μmol/g of defatted air-dried meal.

**11.** The plant of any one of claims 1, 7 or 8, wherein the seeds have an indolyl glucosinolate content of 7.2 μmol/g of defatted air-dried meal.

**12.** The plant of any of claims 1, 7 or 8, wherein the oil has an α-linolenic acid content of 1.7% to 7%.

**13.** The plant of any one of claims 1, 7 or 8, wherein the oil has an α-linolenic acid content of 1.9% to 4.1%.

**14.** The plant of any one of claims 1, 7 or 8, wherein the oil has a sulfur content of 1.3 ppm.

**15.** A fried food product, wherein said food is fried in the canola oil according to claim 4.

**16.** A fried potato product according to claim 15.

**17.** A hydrogenated canola oil obtained by hydrogenating the canola oil according to claim 4.

**Patentansprüche**

**1.** Canola (*Brassica napus*) Pflanze, umfassend
Samen, der einen Gesamtgehalt an Glucosinolaten von 18 μmol oder weniger/g entfettetes, luftgetrocknetes Mehl aufweist,
wobei das sich aus dem Samen ergebende Öl einen α-Linolensäuregehalt von 7% oder weniger im Verhältnis zum Gesamtgehalt an Fettsäuren des Samens und einen Schwefelgehalt von weniger oder gleich 3,0 ppm aufweist, und
die Pflanze zu einer Linie gehört, in der diese Eigenschaften für mindestens drei Generationen stabilisiert sind.

**2.** Samen, hergestellt durch die Pflanze nach Anspruch 1.

**3.** Samen, hergestellt durch die Pflanze nach Anspruch 1, wobei der Gesamtgehalt an Glucosinolaten 15 μmol oder weniger/g entfettetes, luftgetrocknetes Mehl ist.

**4.** Öl des von der Pflanze nach Anspruch 1 hergestellten Samens.

**5.** *Brassica napus* Pflanze, wobei mindestens ein Eltemteil die Pflanze nach Anspruch 1 war.

**6.** Nachkommenschaft der Pflanze nach Ansprüchen 1 oder 5.

**7.** Pflanze, hergestellt durch das Kreuzen einer Canola (*Brassica napus*) Pflanze nach Anspruch 1 mit einer agronomischen Elite-Varietät von *Brassica napus*,
wobei die Pflanze einen Samen mit einem Gesamtgehalt an Glucosinolaten von 18 μmol oder weniger/g entfettetes, luftgetrocknetes Mehl und ein extrahierbares Öl, das (1) einen α-Linolensäuregehalt von 7% oder weniger im Verhältnis zum Gesamtgehalt an Fettsäuren des Samens und (2) einen Schwefelgehalt von weniger als oder gleich 3,0 ppm aufweist, hervorbringt.

**8.** Pflanze nach Anspruch 7, wobei das agronomische Elite-Elternteil die kanadische Canola-Linie Westar ist.

**9.** Pflanze nach einem der Ansprüche 1, 7 oder 8, wobei die Samen einen Gesamtgehalt an Glucosinolaten von 12,5 μmol oder weniger/g entfettetes, luftgetrocknetes Mehl aufweisen.

**10.** Pflanze nach einem der Ansprüche 1, 7 oder 8, wobei die Samen einen aliphatischen Glucosinolatgehalt von 3,7 μmol/g zu 6,3 μmol/g entfettetes, luftgetrocknetes Mehl aufweisen.

**11.** Pflanze nach einem der Ansprüche 1, 7 oder 8, wobei die Samen einen Indolylglucosinolatgehalt von 7,2 μmol/g entfettetes, luftgetrocknetes Mehl aufweisen.

**12.** Pflanze nach einem der Ansprüche 1, 7 oder 8, wobei das Öl einen $\alpha$-Linolensäuregehalt von 1,7% bis 7% aufweist.

**13.** Pflanze nach einem der Ansprüche 1, 7 oder 8, wobei das Öl einen $\alpha$-Linolensäuregehalt von 1,9% bis 4,1% aufweist.

**14.** Pflanze nach einem der Ansprüche 1, 7 oder 8, wobei das Öl einen Schwefelgehalt von 1,3 ppm aufweist

**15.** Gebratenes Lebensmittelprodukt, wobei das Lebensmittel in dem Canolaöl nach Anspruch 4 gebraten ist.

**16.** Gebratenes Kartoffelerzeugnis nach Anspruch 15.

**17.** Gehärtetes Canolaöl, welches durch das Härten des Canolaöls nach Anspruch 4 erhalten wird.

**Revendications**

**1.** Plant de colza (*Brassica napus*) comprenant :

une semence ayant une teneur totale en glucosinolates de 18 μmoles ou moins par gramme de farine dégraissée séchée à l'air ;
la semence produisant une huile ayant une teneur en acide $\alpha$-linolénique de 7 % ou moins par rapport à la teneur totale en acides gras de ladite semence et une teneur en soufre inférieure ou égale à 3,0 ppm ; et
le plant appartenant à une lignée dans laquelle ces propriétés ont été stabilisées pendant au moins trois générations.

**2.** Semence produite par le plant de la revendication 1.

**3.** Semence produite par le plant de la revendication 1, dans laquelle la teneur totale en glucosinolates est de 15 μmoles ou moins par gramme de farine dégraissée séchée à l'air.

**4.** Huile de la semence produite par le plant de la revendication 1.

**5.** Plant de *Brassica napus*, dans lequel au moins un parent est le plant de la revendication 1.

**6.** Lignée du plant de la revendication 1 ou 5.

**7.** Plant produit à partir du croisement d'un plant de colza (*Brassica napus*) selon la revendication 1 avec une variété élite au plan agronomique de *Brassica napus*, le plant produisant une semence ayant une teneur totale en glucosinolates de 18 μmoles ou moins par gramme de farine dégraissée séchée à l'air et une huile extractible ayant (1) une teneur en acide $\alpha$-linolénique de 7 % ou moins par rapport à la teneur totale en acides gras de ladite semence et (2) une teneur en soufre inférieure ou égale à 3,0 ppm.

**8.** Plant selon la revendication 7, dans lequel le parent élite au plan agronomique est la lignée de colza canadien Westar.

9. Plant selon l'une quelconque des revendications 1, 7 ou 8, dans lequel les semences ont une teneur totale en glucosinolates de 12,5 μmoles ou moins par gramme de farine dégraissée séchée à l'air.

10. Plant selon l'une quelconque des revendications 1, 7 ou 8, dans lequel les semences ont une teneur en glucosinolates aliphatiques de 3,7 μmoles à 6,3 μmoles par gramme de farine dégraissée séchée à l'air.

11. Plant selon l'une quelconque des revendications 1, 7 ou 8, dans lequel les semences ont une teneur en glucosinolate d'indolyle de 7,2 μmoles par gramme de farine dégraissée séchée à l'air.

12. Plant selon l'une quelconque des revendications 1, 7 ou 8, dans lequel l'huile a une teneur en acide $\alpha$-linolénique de 1,7 % à 7 %.

13. Plant selon l'une quelconque des revendications 1, 7 ou 8, dans lequel l'huile a une teneur en acide $\alpha$-linolénique de 1,9 % à 4,1 %.

14. Plant selon l'une quelconque des revendications 1, 7 ou 8, dans lequel l'huile a une teneur en soufre de 1,3 ppm.

15. Produit alimentaire frit, dans lequel ledit produit alimentaire est frit dans l'huile de colza selon la revendication 4.

16. Produit frit à base de pomme de terre selon la revendication 15.

17. Huile de colza hydrogénée obtenue en hydrogénant l'huile de colza selon la revendication 4.